(19) Europäisches Patentamt
European Patent Office
Office européen des brevets

(11) EP 3 097 229 B1

(12) EUROPEAN PATENT SPECIFICATION

(45) Date of publication and mention of the grant of the patent:
**05.12.2018 Bulletin 2018/49**

(21) Application number: **15701335.0**

(22) Date of filing: **23.01.2015**

(51) Int Cl.:
***D06M 16/00*** *(2006.01)* ***A01N 63/04*** *(2006.01)*
***C12N 9/18*** *(2006.01)* ***D06M 101/32*** *(2006.01)*

(86) International application number:
**PCT/EP2015/051316**

(87) International publication number:
**WO 2015/110562 (30.07.2015 Gazette 2015/30)**

(54) **A METHOD TO PRODUCE AN ANTIMICROBIAL POLYESTER TEXTILE USING CUTINASE**

VERFAHREN ZUR HERSTELLUNG EINES ANTIMIKROBIELLEN POLYESTER-TEXTILS MITTELS EINER CUTINASE

PROCÉDÉ DE PRODUCTION D'UN TEXTILE POLYESTER ANTIMICROBIEN AU MOYEN D'UNE CUTINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.01.2014 PCT/CN2014/071515**

(43) Date of publication of application:
**30.11.2016 Bulletin 2016/48**

(73) Proprietor: **Novozymes A/S**
**2880 Bagsvaerd (DK)**

(72) Inventors:
- **LAI, Weijian**
  **DK-2880 Bagsvaerd (DK)**
- **LI, Haijing**
  **DK-2880 Bagsvaerd (DK)**
- **SUN, Ting**
  **DK-2880 Bagsvaerd (DK)**
- **ZHOU, Yucheng**
  **DK-2880 Bagsvaerd (DK)**
- **DE MARIA, Leonardo**
  **DK-1807 Frederiksberg (DK)**

(74) Representative: **NZ EPO Representatives**
**Krogshoejvej 36**
**2880 Bagsvaerd (DK)**

(56) References cited:
**EP-A1- 2 476 798**
**WO-A1-2014/012506**
**US-A1- 2002 066 144**
**US-A1- 2002 123 123**
**US-B1- 6 815 190**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Novozymes A/S

<120> A METHOD OF TREATING POLYESTER TEXTILE

<130> 12616-WO-PCT[2]

<160> 1

<170> PatentIn version 3.5

<210> 1
<211> 194
<212> PRT
<213> Humicola insolens

<400> 1

```
Gln Leu Gly Ala Ile Glu Asn Gly Leu Glu Ser Gly Ser Ala Asn Ala
1               5                   10                  15

Cys Pro Asp Ala Ile Leu Ile Phe Ala Arg Gly Ser Thr Glu Pro Gly
            20                  25                  30

Asn Met Gly Ile Thr Val Gly Pro Ala Leu Ala Asn Gly Leu Glu Ser
        35                  40                  45

His Ile Arg Asn Ile Trp Ile Gln Gly Val Gly Gly Pro Tyr Asp Ala
    50                  55                  60

Ala Leu Ala Thr Asn Phe Leu Pro Arg Gly Thr Ser Gln Ala Asn Ile
65                  70                  75                  80

Asp Glu Gly Lys Arg Leu Phe Ala Leu Ala Asn Gln Lys Cys Pro Asn
                85                  90                  95

Thr Pro Val Val Ala Gly Gly Tyr Ser Gln Gly Ala Ala Leu Ile Ala
            100                 105                 110

Ala Ala Val Ser Glu Leu Ser Gly Ala Val Lys Glu Gln Val Lys Gly
        115                 120                 125

Val Ala Leu Phe Gly Tyr Thr Gln Asn Leu Gln Asn Arg Gly Gly Ile
    130                 135                 140

Pro Asn Tyr Pro Arg Glu Arg Thr Lys Val Phe Cys Asn Val Gly Asp
145                 150                 155                 160

Ala Val Cys Thr Gly Thr Leu Ile Ile Thr Pro Ala His Leu Ser Tyr
                165                 170                 175

Thr Ile Glu Ala Arg Gly Glu Ala Ala Arg Phe Leu Arg Asp Arg Ile
            180                 185                 190

Arg Ala
```

SEQUENCE LISTING

<110> Novozymes A/S

<120> A METHOD OF TREATING POLYESTER TEXTILE

<130> 12616-WO-PCT[2]

<160> 1

<170> PatentIn version 3.5

<210> 1
<211> 194
<212> PRT
<213> Humicola insolens

<400> 1

```
Gln Leu Gly Ala Ile Glu Asn Gly Leu Glu Ser Gly Ser Ala Asn Ala
1               5                   10                  15

Cys Pro Asp Ala Ile Leu Ile Phe Ala Arg Gly Ser Thr Glu Pro Gly
            20                  25                  30

Asn Met Gly Ile Thr Val Gly Pro Ala Leu Ala Asn Gly Leu Glu Ser
        35                  40                  45

His Ile Arg Asn Ile Trp Ile Gln Gly Val Gly Gly Pro Tyr Asp Ala
    50                  55                  60

Ala Leu Ala Thr Asn Phe Leu Pro Arg Gly Thr Ser Gln Ala Asn Ile
65                  70                  75                  80

Asp Glu Gly Lys Arg Leu Phe Ala Leu Ala Asn Gln Lys Cys Pro Asn
                85                  90                  95

Thr Pro Val Val Ala Gly Gly Tyr Ser Gln Gly Ala Ala Leu Ile Ala
            100                 105                 110

Ala Ala Val Ser Glu Leu Ser Gly Ala Val Lys Glu Gln Val Lys Gly
        115                 120                 125

Val Ala Leu Phe Gly Tyr Thr Gln Asn Leu Gln Asn Arg Gly Gly Ile
    130                 135                 140

Pro Asn Tyr Pro Arg Glu Arg Thr Lys Val Phe Cys Asn Val Gly Asp
145                 150                 155                 160

Ala Val Cys Thr Gly Thr Leu Ile Ile Thr Pro Ala His Leu Ser Tyr
                165                 170                 175

Thr Ile Glu Ala Arg Gly Glu Ala Ala Arg Phe Leu Arg Asp Arg Ile
            180                 185                 190

Arg Ala
```

SEQUENCE LISTING

<110> Novozymes A/S

<120> A METHOD OF TREATING POLYESTER TEXTILE

<130> 12616-WO-PCT[2]

<160> 1

<170> PatentIn version 3.5

<210> 1
<211> 194
<212> PRT
<213> Humicola insolens

<400> 1

```
Gln Leu Gly Ala Ile Glu Asn Gly Leu Glu Ser Gly Ser Ala Asn Ala
1               5                   10                  15

Cys Pro Asp Ala Ile Leu Ile Phe Ala Arg Gly Ser Thr Glu Pro Gly
            20                  25                  30

Asn Met Gly Ile Thr Val Gly Pro Ala Leu Ala Asn Gly Leu Glu Ser
        35                  40                  45

His Ile Arg Asn Ile Trp Ile Gln Gly Val Gly Gly Pro Tyr Asp Ala
    50                  55                  60

Ala Leu Ala Thr Asn Phe Leu Pro Arg Gly Thr Ser Gln Ala Asn Ile
65                  70                  75                  80

Asp Glu Gly Lys Arg Leu Phe Ala Leu Ala Asn Gln Lys Cys Pro Asn
                85                  90                  95

Thr Pro Val Val Ala Gly Gly Tyr Ser Gln Gly Ala Ala Leu Ile Ala
            100                 105                 110

Ala Ala Val Ser Glu Leu Ser Gly Ala Val Lys Glu Gln Val Lys Gly
        115                 120                 125

Val Ala Leu Phe Gly Tyr Thr Gln Asn Leu Gln Asn Arg Gly Gly Ile
    130                 135                 140

Pro Asn Tyr Pro Arg Glu Arg Thr Lys Val Phe Cys Asn Val Gly Asp
145                 150                 155                 160

Ala Val Cys Thr Gly Thr Leu Ile Ile Thr Pro Ala His Leu Ser Tyr
                165                 170                 175

Thr Ile Glu Ala Arg Gly Glu Ala Ala Arg Phe Leu Arg Asp Arg Ile
            180                 185                 190

Arg Ala
```

## Description

### REFERENCE TO SEQUENCE LISTING

This application contains a Sequence Listing in computer readable form. The computer readable form is incorporated herein by reference.

### FIELD OF THE INVENTION

The present invention relates to an antimicrobial method on polyester textile by contacting polyester textile with a cutinase in an aqueous solution, as well as a textile produced by such method.

### BACKGROUND OF THE INVENTION

Polyethylene terephthalate (abbreviated as PET) fibers accounts for the main part of the polyester applied by the textile industry. PET fiber has certain key advantages including high strength, soft hand, stretch resistance, stain resistance, machine washability, wrinkle resistance and abrasion resistance.

To date, many composite PET fibers have been researched to improve PET fibers' properties such as thermal and crystalline or introduce new functional properties such as electrical conductibility, antibacterial function, and flame-retardant to the fibers (Journal of Applied Polymer Science, Vol. 112, 1927-1932 (2009)). For synthetic fibers, the antimicrobial active agents can be incorporated into the polymer prior to extrusion or blended into the fibers during their formation.

In recent years, antimicrobial finishing of textiles has become extremely important in the production of protective, decorative and technical textile products. Antimicrobial finishing of textiles protects users from pathogenic or odor-generating microorganisms, which can cause medical and hygienic problems, and protects textiles from undesirable aesthetic changes or damage caused by rotting, which can result in reduced functionality. As a consequence of their importance, the number of different antimicrobial agents suitable for textile application on the market has increased dramatically.

Antimicrobial agents either inhibit the growth (-static) or kill (-cidal) the microorganisms. Almost all antimicrobial agents used in commercial textiles, e.g. silver, triclosan, polyhexamethylene biguanide (PHMB) and quaternary ammonium compounds, are biocides. However, their attachment to a textile surface or incorporation within the fiber substantially reduces their activity and limits their availability. Furthermore, the biocide can be gradually lost during the use and washing of the textile. For these reasons, large amounts of these biocides need to be applied to textiles to effectively control bacterial growth and to sustain durability (Textile Research Journal Vol 78(1): 60-72).

WO 1999/001604 discloses a method of reducing the pilling propensity of polyester fabrics and/or garments with a terephtalic acid diethyl ester hydrolytic enzyme (ETE hydrolytic enzyme) and/or an ethyleneglycol dibenzyl ester hydrolytic enzyme (BEB hydrolytic enzyme).

WO 2001/092502 discloses the treatment of polyester textile with *Humicola insolens* cutinase variants.

WO 2014/012506 discloses methods to reduce pilling, biopolish, improve the wettability/hydrophilicity, and confer anti-static properties to polyester textile by contacting polyester textile with a cutinase and glycosyl hydrolase family 61 in an aqueous solution.

US 6815190 discloses cutinase variants having improved thermostability and use thereof, e.g. for reduction of malodor in polyester textile.

US 2002/0066144 discloses a method to prevent back staining of dyes during stone washing of polyester textile by contacting polyester textile with a cutinase in an aqueous solution.

EP 2476798 discloses a method to confer antifouling properties to textile materials in aquatic environments. Textiles such as polyamide are treated with enzymes such as peptidase.

Customer's desire for comfort, hygiene and well-being has created a large and rapidly increasing market for antimicrobial textiles. Numerous manufacturers in the textile industry have responded to this demand by launching their brands of antimicrobial products. There is always a need in textile industry for a better antimicrobial solution.

### SUMMARY OF THE INVENTION

The present invention relates to an antimicrobial method on polyester textile by contacting polyester textile with a cutinase in an aqueous solution.

In some embodiments, the polyester textile is a PET textile.

In some embodiments, the present invention also relates to a textile composition comprising a glycosyl hydrolase family 61 polypeptide and a cutinase.

In some embodiments, the polyester textile treatment process may further comprise one or more enzymes selected from the group consisting of lipases, esterases, laccases, peroxidases and peroxygenase and transferases.
In some embodiments, the microbe is bacteria.
In some embodiments, the method for manufacturing polyester textile is provided. In some embodiments, the textile is manufactured from fabric to garment.

## DETAILED DESCRIPTION OF THE INVENTION

The invention will now be described in detail by way of reference using the following definitions and examples.
As used herein, the singular terms "a", "an," and "the" include the plural reference unless the context clearly indicates otherwise.

### Polyester Textile

"Polyester" as used herein means a linear polymeric molecule containing in-chain ester groups which are derived from condensation of a diacid with a diol or from the polymerization of hydroxy acids. The present invention applies to both aliphatic and aromatic polyesters.
Particularly preferred polyesters are aromatic polyester articles which are used to produce fiber and resin and that comprise a synthetically produced long chain polymer comprising at least 85%, preferably at least 90% and most preferably at least 95%, by weight of an ester of a substituted aromatic carboxylic acid, such as substituted terephthalic acid or parasubstituted hydroxybenzoate or a mixture thereof. Other useful polyester articles include those made of bulk polymer, yarns, fabrics, films, resins and powders. The principal polyesters in industrial usage include polyethylene terephthalate (PET), tetramethylene terephthalate (PTMT), polybutylene terphthalate (PBT), polytrimethylene terephthalate (PTT) and polyethylenenaphthalate (PEN), polycyclohexanedimethylene terephthalate (CHDMT), polyethylene-4-oxybenzoate, A-Tell, polyglycolide, PHBA and 2GN. However, PET is the most common linear polymer produced and accounts for a majority of the polyester applied in industry today.
The polyester textile used herein is meant to include fibers, yarns, fabrics and garments comprising polyester. The polyester yarn or fabric or garment is made from pure poly (ethylene terephthalate), or is made from blends of poly (ethylene terephthalate) fibers and any other materials conventionally used for making textile such as wool, cotton, viscose and silk.
In a preferred embodiment the polyester fabric is a fabric blend comprising more than 5% (w/w) of polyester, in particular more than 10%, more than 15%, more than 20%, more than 30%, more than 35%, more than 50%, more than 65%, more than 90%, or more than 95% of polyester. In an even preferred embodiment, such fabric blend is polyester/cotton blend fabric. In an even preferred embodiment, the process of the invention is applied to textile consisting essentially of polyester, i.e. pure polyester textile (100% polyester), such as pure PET textile.

### Cutinase

Cutinases are lipolytic enzymes classified as EC 3.1.1.74 according to Enzyme Nomenclature. Reference is made to the Recommendations of the Nomenclature Committee of the International Union of Biochemistry and Molecular Biology, Academic Press Inc., 1992.
For purposes of the present invention, cutinase activity is determined using oligomer Terephtalic acid-bis-2-benzoyloxy-ethylesther (BETEB) as substrate according to Method section in the Example of the present application. BETEB is a by-product during the PET synthesis and is generally remained in the fabric or garment during textile manufacturing. BETEB is produced by e.g. condensation of terephthalic acid, benzoic acid and ethylene glycol, which has the same unit of benzoyloxy-ethylester as PET.
The enzyme in question qualifies as a cutinase for use according to the present invention if transparent zones are shown after testing.
Cutinases are known from various fungi, such as a filamentous fungal cutinase, e.g. native to a strain of *Humicola* or *Fusarium or Magnaporthe or Pseudomonas,* specifically H. *insolens* or *F. solani pisi or Magnaporthe grisea or Pseudomonas mendocina,* more specifically *H. insolens* strain DSM 1800 (US 5,827,719), or *F. solani pisi* (WO 90/09446 Fig 1; WO 94/14964 Fig1D, WO 94/03578 Fig 1 D) or *Magnaporthe grisea* (WO10/107560 SEQ ID NO: 1) or *Pseudomonas mendocina* ATCC 53552 (US 5,389,536, claim 1).
SEQ ID NO: 1 is the amino acid sequence of the *Humicola insolens* cutinase (corresponding to the mature part of SEQ ID NO: 2 of US 5,827,719).
In some embodiments, the cutinase is variants comprising a substitution, deletion, and/or insertion of one or more (or several) amino acids of SEQ ID NO: 1. Preferably, the total number of amino acid substitutions, deletions and/or insertions of the SEQ ID NO: 1 is not more than 10, *e.g.,* 1, 2, 3, 4, 5, 6, 7, 8 or 9. The *Humicola insolens* cutinase

variants are described in WO 2001/092502 The cutinase enzyme may also be a variant of a parent cutinase such as those described in WO 00/34450.

In some embodiments, the cutinase variant has at least 70%, or 75%, or 85%, or 90%, or 95%, or 96%, or 97%, or 98%, or 99%, or 100% identity to SEQ ID NO: 1.

The fungal cutinase may also be derived from other fungal strains such as a strain of *Rhizoctonia,* e.g. *R. solani,* or a strain of *Alternaria,* e.g. *A. brassicicola* (WO 94/03578).

Preferably the cutinase has a pH optimum within 1 pH unit of the pH of the process, e.g. if the processss is run at pH 8, the cutinase preferably has a pH optimum between 7 and 9.

## Variant

The term "variant" means a polypeptide having cutinase activity comprising an alteration, *i.e.,* a substitution, insertion, and/or deletion, at one or more (e.g., several) positions. A substitution means replacement of the amino acid occupying a position with a different amino acid; a deletion means removal of the amino acid occupying a position; and an insertion means adding an amino acid adjacent to and immediately following the amino acid occupying a position. The variants of the present invention have at least 20%, at least 30%, at least 40%, at least 50%, at least 55%, at least 60%, at least 65%, at least 70%, at least 75%, at least 80%, at least 85%, at least 90%, at least 95%, or at least 100% of the cutinase activity of the mature polypeptide of SEQ ID NO: 1

In describing the variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), e.g., "Gly205Arg +Ser411Phe" or "G205R +S411F", representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), e.g., "Gly195* +Ser411*" or "G195* +S411*".

Different alterations. Where different alterations can be introduced at a position, the different alterations are separated by a comma, e.g., "Arg170Tyr,Glu" represents a substitution of arginine at position 170 with tyrosine or glutamic acid. Thus, "Tyr167Gly,Ala +Arg170Gly,Ala" designates the following variants: "Tyr167Gly+Arg170Gly", "Tyr167Gly +Arg170Ala", "Tyr167Ala +Arg170Gly", and "Tyr167Ala +Arg170Ala".

## Preparation of Variants

The variants can be prepared using any mutagenesis procedure known in the art, such as site-directed mutagenesis, synthetic gene construction, semi-synthetic gene construction, random mutagenesis, shuffling, etc.

Site-directed mutagenesis is a technique in which one or more (e.g., several) mutations are introduced at one or more defined sites in a polynucleotide encoding the parent.

Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, *e.g.,* Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.,* US2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA

86: 2152-2156; WO95/17413; or WO95/22625. Other methods that can be used include error-prone PCR, phage display (*e.g.*, Lowman et al., 1991, Biochemistry 30: 10832-10837; US5223409; WO92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide subsequences may then be shuffled.

## Sequence Identity

The relatedness between two amino acid sequences or between two nucleotide sequences is described by the parameter "sequence identity".

For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et a/., 2000, supra), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

## Glycoside hydrolase family 61 (GH61) polypeptides

The term "glycoside hydrolase family 61" or "GH61" is defined herein as a polypeptide falling into the glycoside hydrolase family 61 according to Henrissat B., 1991, Biochem. J. 280: 309-316, and Henrissat B., and Bairoch A., 1996, Biochem. J. 316: 695-696.

The present invention relates to the use of isolated GH61 polypeptides in general. A GH61 polypeptide useful in the present invention may be obtained from microorganisms of any genus. For purposes of the present invention, the term "obtained from" as used herein in connection with a given source shall mean that the polypeptide encoded by a nucleotide sequence is produced by the source in which it is naturally present or by a strain in which the nucleotide sequence from the source has been inserted. In a preferred aspect, the polypeptide obtained from a given source is secreted extracellularly.

A GH61 polypeptide of the present invention may be a bacterial polypeptide. For example, the polypeptide may be a gram positive bacterial polypeptide such as a *Bacillus* polypeptide, e.g., a *Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis,* or *Bacillus thuringiensis* polypeptide; or a *Strepto-*

*myces* polypeptide, e.g., a *Streptomyces lividans* or *Streptomyces murinus* polypeptide; or a gram negative bacterial polypeptide, e.g., an E. *coli* or a *Pseudomonas* sp. polypeptide.

A GH61 polypeptide of the present invention may also be a fungal polypeptide, and more preferably a yeast polypeptide such as a *Candida, Kluyveromyces, Pichia, Saccharomyces, Schizosaccharomyces,* or *Yarrowia* polypeptide; or more preferably a filamentous fungal polypeptide such as an *Acremonium, Aspergillus, Aureobasidium, Chaetomium, Cryptococcus, Filibasidium, Fusarium, Humicola, Magnaporthe, Mucor, Myceliophthora, Neocallimastix, Neurospora, Paecilomyces, Penicillium, Piromyces, Poronia, Schizophyllum, Talaromyces, Thermoascus, Thielavia, Tolypocladium, Trichoderma* or *Verticillium* polypeptide.

In the present invention, any GH61 polypeptide having cutinase enhancing activity can be used.

## Antimicrobial Efficacy

The method of present invention results in the antimicrobial efficacy. The term "antimicrobial" means inhibit or reduce the microbe grown on the PET fabric or PET/Cotton blend fabric. The microbe on the fabric can be bacterial or fungi.

In some embodiments, the method of the present invention inhibits the bacterial growth on the PET fabric or PET/Cotton blend fabric.

A number of test methods have been developed to determine the efficacy of antimicrobial on fabric. The bacterial species *Klebsiella Pneumoniae* is recommended in most test methods. The species is potentially pathogenic and therefore requires proper physical containment facilities for handling (e.g. a biosafety cabinet). Many studies have used the innocuous *Escherichia coli* as a test microorganism which can be cultured and handled in a standard laboratory with minimal health risk.

For the purpose of the present invention, the antimicrobial efficacy is measured according to any one of the standard test method ASTM E 2149-01 (American Society for Testing and Materials), testing Method AATCC 100-2004 (American Association of Textile Chemists and Colorists), and Agar plate test method as Example 4. The antimicrobial efficacy shows that the bacteria grown in the PET fabric are reduced compared with the bacteria grown on the PET fabric without cutinase treatment.

## Polyester Fabric Manufacturing Process

Polyester such as poly (ethylene terephthalate) is synthesized by condensation, drawn into fibers from a melt, possibly cut to stables, possibly mixed with other fiber types, and spun to yarn.

After yarn is knitted or woven into fabric, the fabric is normally treated to remove spin finish oil, for example in a process where the fabric will first be heat set at 180°C and then be pretreated with surfactants (sometimes also with addition of alkali) at 80-100°C and then optionally followed by the weight reduction process by using severe alkali at up to 130°C to hydrolyze polyester fabric to make it more soft and luster appearance. Then the polyester fabric will be heat set and dyed with disperse dyestuffs at pH 4.5-6 at up to 130°C, followed by reduction clearing with sodium hyposulphite at 60-80°C, pH 10. If necessary, these processes can be followed by finishing (post treatment) steps to further improve the textile properties, such as anti-pilling, wettability improvement or anti-static treatment. For PET/cotton blend fabric, there will be a soaping step after finishing.

The method of the present invention of the antimicrobial method on polyester textile by contacting polyester textile with a cutinase in an aqueous solution takes place during one or more of the subsequent steps of pretreatment, weight reduction, disperse dyeing, post finishing and soaping step. The method of the present invention may take place either as a separate step or in combination with any of the existing polyester processing steps.

In some embodiments, it has been optimized to make antimicrobial method integrated into the finishing and dyeing process of polyester blends and maintain the highest performance. Cellulase and cutinase can be combined in reactive dyeing process; cellulase and cutinase can be combined in one bath of soaping step; separately applied with cellulase treatment combined in reactive dyeing process, cutinase treatment in one bath of soaping or after soaping.

The process of the invention is readily applicable in the textile industry as it can be carried out using existing wet processing apparatus, such as in a jet dyer, a Pad-Roll, a Jigger/Winch, a J-Box, or Pad-Steam types of apparatus. The process preferably takes place during the finishing (post treatment) step.

## PROCESS CONDITION

Cutinase can be used during polyester textile manufacturing process, either as a separate step after any of the existing polyester manufacturing steps, or in combination with any of the existing polyester manufacturing steps like pretreatment, weight reduction, disperse dyeing, post finishing or soaping.

It is advised that a suitable liquor/textile ratio to be used in the present method may be in the range of from about 20:1 to about 1:1, preferably in the range of from about 15:1 to about 3:1, more preferably in the range of from

15:1 to 5:1 (Volumn/weight, ml/g).

The reaction time for the present invention is usually in the range of from about 10 minutes to about 8 hours. Preferably the reaction time is within the range of from about 20 minutes to about 180 minutes, more preferably the reaction time is within the range of from about 30 minutes to about 150 minutes, most preferably the reaction time is within the range of from about 45 minutes to about 120 minutes.

The pH of the reaction medium greatly depends on the enzyme(s) in question. Preferably the process of the invention is carried out at +/- 1 pH unit from the pH optimum of the cutinase. Preferably, the process of the invention is carried out at a pH in the range of from about pH 3 to about pH 11, preferably in the range of from about pH 4 to about pH 10, or within the range of from about pH 6 to about pH 9.

The process temperature of the present invention is preferably selected according to the optimal temperature of the cutinase +/- 10°C. Preferably the process is able to function at a temperature below 100°C, preferably below 90°C, more preferably below 80°C, and even more preferably below 75°C.

In some embodiments, the process of the present invention is conducted at the temperature range of 40-100°C, preferably 50-90°C, preferably 60-85°C, more preferably 65-80°C, and even more preferably 70-80°C.

Enzyme dosage greatly depends on the enzyme reaction time, i.e. a relatively short enzymatic reaction time necessitates a relatively increased enzyme dosage, and vice versa. In general, enzyme dosage may be stipulated in accordance with the reaction time available.

The amount of cutinase to be used according to the method of the present invention depends on many factors and should preferably be optimized by the skilled person. According to the present invention the preferred concentration of the cutinase enzyme in the aqueous medium is from about 0.01 to about 50 milligram enzyme protein per gram of polyester textile, preferably 0.05-20 milligram of enzyme protein per gram of polyester textile, more preferably 0.1-15 milligram of enzyme protein per gram of polyester textile, and even more preferably 0.2-5 milligram of enzyme protein per gram of polyester textile.

The process of the invention may optionally comprise a rinsing step during which the hydrolyzed oligomers are subjected to rinsing, in particular to rinse with alkali solution. Alkali solution dissolves linear fragments of the oligomers, and may to some extent further hydrolyze these linear fragments.

The aqueous composition used in the method of the invention may further comprise one or more enzymes selected from the group consisting of lipases, esterases, laccases, peroxidase and etc.

## Composition for treating textile

The present invention also encompasses a composition suitable for treating textile where the composition comprises a cutinase.

The textile composition of the present invention is adapted for one or more of the polyester manufacturing processes such as pretreatment, weight reduction, disperse dyeing and post finishing, either in a separate step or in combination with any of those steps.

In some embodiments of the invention, the composition containing a cutinase further comprises other components, including without limitation other enzymes, as well as one or more of surfactants, bleaching agents, antifoaming agents, builder systems, and the like.

Enzymes suitable for use in the present invention include without limitation lipases, esterases, laccases, peroxidases, peroxygenase and transferases.

The textile composition can be in any form, such as a solid, liquid, paste, gel or any combination thereof.

## Surfactant

In the treatment of polyester textile, a conventional surfactant may be used to improve the contact with the enzyme.

The textile composition of the present invention may comprise one or more surfactants, which may be anionic and/or cationic and/or non-ionic and/or semi-polar and/or zwitterionic, or a mixture thereof. The surfactant(s) is typically present at a level of from about 0.001% to 20% by weight of composition, such as about 0.005% to about 10%, or about 0.01% to about 5%, or about 0.02% to about 1%.

More specifically, the surfactant used in the process or the composition of the present invention comprises a non-ionic surfactant. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), Triton, nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamide (PFAM), polyhydroxy alkyl fatty acid amides, or N-acyl N-alkyl derivatives of glucosamine (glucamides, GA, or fatty acid glucamide, FAGA), as well as products

available under the trade names SPAN and TWEEN, and combinations thereof.

**Other enzymes**

The enzymatic polyester manufacturing process as well as the textile composition may comprise one or more additional enzymes such as a lipase, esterase, laccase, peroxidase, peroxygenase and transferases.

Lipases: Suitable lipases include those of bacterial or fungal origin. Chemically modified or protein engineered mutants are included. The lipase may for example be triacylglycerol lipase (EC3.1.1.3), phospholipase A2 (EC 3.1.1.4), Lysophospholipase (EC 3.1.1.5), Monoglyceride lipase (EC 3.1.1.23), galactolipase (EC 3.1.1.26), phospholipase A1 (EC 3.1.1.32), Lipoprotein lipase (EC 3.1.1.34). Examples include lipase from *Thermomyces, e.g.,* from *T. lanuginosus* (previously named *Humicola lanuginosa*) as described in EP 258 068 and EP 305 216, a *Pseudomonas* lipase, *e.g.*, from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, *e.g.*, from *B. subtilis* (Dartois et al., 1993, Biochemica et Biophysica Acta, 1131: 253-360), B. *stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO2007/087508 and WO 2009/109500.

Preferred commercially available lipase enzymes include Lipolase™, Lipolase Ultra™, and Lipex™; Lecitase™, Lipolex™; Lipoclean™, Lipoprime™ (Novozymes A/S).

Peroxidases/Oxidases: Suitable peroxidases/oxidases include those of plant, bacterial or fungal origin. Chemically modified or protein engineered mutants are included. Examples of useful peroxidases include peroxidases from *Coprinus, e.g.,* from *C. cinereus,* and variants thereof as those described in WO 93/24618, WO 95/10602, and WO 98/15257.

Commercially available peroxidases include Guardzyme™ (Novozymes A/S).

Peroxygenase: The term "peroxygenase" means an "unspecific peroxygenase" activity according to EC 1.11.2.1, that catalyzes insertion of an oxygen atom from $H_2O_2$ into a variety of substrates, such as nitrobenzodioxole. Examples of useful peroxygenase include peroxygenase described in WO 2008/119780.

**Examples**

**Enzymes**

Cellusoft CR® (a mono-component *Thielavia terrestris* GH45 endoglucanase product, commercially available from Novozymes A/S, described in WO96/29397);

Cutinase variant: variant of cutinase from *Humicola Insolens,* with alterations A161L +R181P +G182* on the parent *H. insolens* cutinase of SEQ ID NO: 1 (cutinase variant can be produced according to the method described in WO 2001/092502)

**Material**

LB agar medium: 10g of Trypone (Oxoid, cat# LP0042), 5g of yeast extract (Oxoid, cat# LP0021), 10g of NaCl, 15g of agar, in $dH_2O$ up to 1L, autoclaved at 121°C for 20 mins;

LB agar plate: re-dissolve LB agar medium and cool to 50-60°C, then make aliquot to sterilized petri dish (9cm);

LB medium: 10g of Trypone, 5g of yeast extract, 10g of NaCl, dissolved in $dH_2O$ up to 1L, autoclaved at 121°C for 20mins;

Nutrient agar: Add 1.5% bacteriological agar to nutrient broth. Heat to boiling. Check pH and adjust to 7.1 ± 0.1 using NaOH solution if necessary. Dispense in 15 ± 1 mL amounts in conventional bacteriological culture tubes. Plug and sterilize at 103 kPa (15 psi) for 15 min.

Nutrient broth: Peptone (Bacto-peptone) 5g, Beef extract 3g, Distilled water to 1000 mL.

E. coli: TOP10 (Cat# CB104-02) from TIANGEN Biotech Co. Ltd., Beijing, China

TC blend (polyester/cotton blend): 60% polyester/30% cotton/5% Spandex, 32S, knitted, purchased from Dongcheng Cloth Trading Co. Ltd., China;

100% staple PET: 100% polyester, 21S, single jersey.

**Method**

Weight loss determination

The swatches were placed in the conditioned room (65%+/-5% humidity, 20+/-1°C) for 24 hours before they were numbered, weighed by the analytical balance (for samples below 100g) or a precision balance (for samples over 100g) and recorded. After treatment, all samples were tumbled dried (AEG, LAVATHERM 37700, Germany) for 1 hour and conditioned for 24 hour in conditioned room same as above. For each sample, the weight loss was defined as below:

Weight loss = (weight before treatment - weight after treatment)/weight before treatment X (100%)

Pilling Notes test

Fabrics including treated and untreated which had been pre-conditioned in norm climate (65% humidity, 20°C) for at least 24 hours were tested for the pilling notes with Nu-Martindale Tester (James H. Heal Co. Ltd, England), with untreated fabrics of the same type as the abraded fabrics. A standard pilling test (Swiss Norm (SN) 198525) was carried out after 2000 Revolutions by marking from 1-5, with the meaning defined as below, where 1 shows poor anti-pilling and 5 shows excellent anti-pilling property. Thus the higher the Martindale pilling notes score the more effective the biopolishing treatment.

Note 5: No pilling
Note 4: Slight Pilling
Note 3: Moderate Pilling
Note 2: Distinct Pilling
Note 1: Heavy Pilling
1/2, 1/4 notes are allowed

To make the test result more reliable, 3 separate readings were carried out by different persons for each sample, and the average of the 3 readings was adopted as the final result of pilling notes.

Protein Content

The enzyme protein in an enzyme product can be measured with BCA™ Protein Assay Kit (product number 23225, commercial available from Thermo Fisher Scientific Inc.) according to the product manual.

BETEB-Agar plate for evaluation of the cutinase activity

BETEB was hydrolyzed by cutinase into more soluble agents. Thus, after hydrolysis by enzyme, there were transparent zones on the plates poured with the mixture of Agar and BETEB.

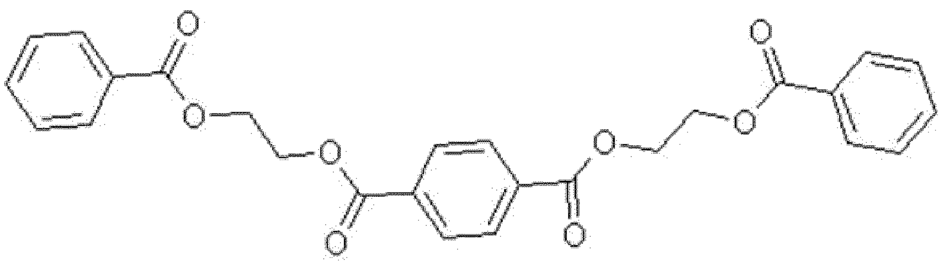

BETEB molecule structure

Hydrolysis of BETEB will produce

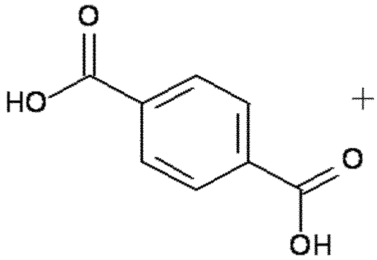
+
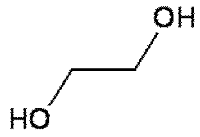
+
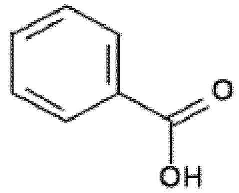

Cutinase activity was measured by the below process:

a) BETEB solution preparation: 5 ml 100% ethanol was added into a glass bottle with a plug, 20 mg BETEB was added into the ethanol and then the bottle was placed in a 60°C water bath to dissolve the BETEB.
b) 1.5% agar solution was prepared by adding 0.75 g agar into 45 ml Tris-HCl buffer (25mM, pH 7.0), and then placing the baker in a Microwave oven heating twice for 30 seconds to dissolve the Agar.
c) The agar solution was cooled down to 60°C and mixed with the BETEB solution prepared in step a. The mixture was poured into a petri dish.
d) Small holes were dug in the petri dish with a tip of 6 mm diameter or puncher.
e) Enzyme sample of 30 microgram /ml was added into the petri dish by a tip with 75 microliter (ul) enzyme sample for each hole. The petri dish was placed at 37°C overnight.

Cutinase variant showed transparent zones in the area around the holes, as BETEB was hydrolyzed by the cutinase.

**Example 1. Pretreatment with cellulase/cutinase in Launder-O-Meter**

The fabric treatments done in Launder-O-Meter (LOM, SDL-Atlas LP2) included: cellulase treatment (for TC blend); and/or cutinase treatment (for both TC blend and staple PET).

Cellulase treatment

The TC blend fabrics were cut into rectangular pieces with 16 cm tall and 16 cm long and weight about 4-5 g. The fabrics were placed in the conditioned room (65% relative humidity, 20°C) for 24 hours before they were numbered, weighed by the analytical balance and recorded. Two conditioned piece were placed in each beaker. For each beaker, 20 steel balls (about 220g in total) were used to supply the mechanical aids. Then the pH 6.5 buffer (5.642g $Na_2HPO_4 \cdot 12H_2O$ and 5.344 g $NaH_2PO_4 \cdot 2H_2O$ in 1 L de-ionized water) and the cellulase solution or buffer were added according to Table 1, based on the calculation of actual fabric weights, with a liquid to fabric ratio of 10:1 (v/w).

The LOM machine was started after the required program was chosen, and it would hold when the temperature reached 55°C. Each beaker was fitted with a lid lined with 2 neoprin gaskets and close tightly with the metal clamping device. The beakers were loaded into the preheated LOM. Metal racks were used to accommodate and secure 5 beakers, in the vertical position, in each of the 4 drum positions. The LOM lid was closed and the washing program was continued and the timing was initiated. 1 hour later, all beakers were removed and the fabric samples were transferred to the inactivation solution (2g/L sodium carbonate) at 85°C for 10 minutes. Then the fabrics were rinsed in hot water for 2 times and in cold water for 2 times. The fabrics were tumble-dried (AEG, LAVATHERM 37700, Germany) for 1 hour, and then the samples were conditioned for 24 hours at 20°C, 65% relative humidity prior to evaluation. The fabrics were sent to external agent to assess their anti-microbial performance according to the industry standards as in Example 3.

Cutinase treatment

The staple PET fabrics were cut in a similar way as above and some TC blend fabric treated with cellulase were placed in the conditioned room before they were treated in LOM.

Cutinase treatment process in LOM was the same as the cellulase treatment except the followings: in each beaker one piece of fabric and 10 steel balls (about 110g in total) were loaded; the buffer of pH 8 (2g/L $Na_2CO_3$ adjusted with acetate acid) containing 0.2g/L Triton X-100 was used here; the treatment condition 8was 70°C for 2 hours; the treatment solution used here was cutinase or pH 8 buffer as in Table 1.

Table 1 shows that cellulase and/or cutinase impoves the pilling note and increases the weight loss which means that both cellulase and cutinase react with the fabric under the conditions specified in Example 1.

Table 1. Pretreatment of PET containing fabrics in LOM

| Fabric No. | Fabric type | Cellulase treatment | Cutinase treatment | Weight loss(%) | Pilling notes |
|---|---|---|---|---|---|
| 1 | TC blend | No | No | 0 | 1.5 |
| 2 | | buffer | buffer with X-100 | 0.1 | 1.5 |
| 3 | | 1.2 g/L Cellusoft CR | No | 2.3 | 2.2 |
| 4 | | 1.2 g/L Cellusoft CR | 0.4 mg PETHG0185/g fabric | 2.7 | 2.8 |
| 5 | 100% staple PET | No | No | 0 | 1.5 |
| 6 | | No | buffer with X-100 | 0.35 | 1.5 |
| 7 | | No | 0.4 mg PETHG0185/g fabric | 0.8 | 2.5 |

**Example 2 Pretreatment with NaOH in Lab-O-Mat**

It is a well-known process in textile industry to use NaOH to treat PET, aiming for improved physical properties of fabrics, such as better wettability, air-permeability and also a silk like appearance. It is a hydrolysis reaction between NaOH in the solution and the PET fabric. But in this Example we would like to see whether this treatment will lead to anti-bacterial effects or not.

The 100% staple PET was cut as Example 1 and treated in Lab-O-Mat (Mathis) to achieve weight reduction. In each beaker, one fabric was loaded with 20 g/L NaOH solution to get a liquor ratio 1:20 (w/v) and then treated with Lab-O-Mat at 100°C for 60 min before the beakers were cooled down to 70°C and the fabrics were rinsed with hot water (about 50°C) and cold water (about 25°C) and neutralized with acetate acid. The fabrics were then centrifuged and tumble-dried as Example 1. The resulting fabrics were also sent for anti-microbial test as in Example 3.

Table 2. Pretreatment of 100% staple PET in Lab-O-Mat

| Fabric No. | Fabric type | NaOH treatment | Weight loss(%) | Pilling notes |
|---|---|---|---|---|
| 8 | 100% staple PET | 20g/L NaOH | 4.9 | 2.5 |

**Example 3 Anti-microbial performance test by Intertek**

The fabrics resulted from Example 1 and 2 were sent to Intertek for anti-microbial performance according to standard test method of American Society for Testing and Materials ASTM E 2149-01 under dynamic contact conditions or Test Method AATCC 100-2004 (American Association of Textile Chemists and Colorists).

For ASTM E 2149-01 based tests, the test conditions were as below:

*Klebsiella Pneumoniae* (ATCC 4352) was inoculated in LB medium. The incubated test culture in a nutrient broth was diluted with a sterilized phosphate buffer (pH 7.2) to give a concentration of 1.5-3.0 x $10^5$ CFU/ml (working dilution). Each fabric (about 1 g) was transferred to flask containing 50 ml of the working dilution. All flasks were shaken for 1 hour at 300 rpm. After a series of dilutions of the bacterial solutions using the buffer solution, 1 ml of the solution was plated in nutrient agar. The inoculated plates were incubated at 37°C for 24-48 hours and the surviving cells were counted. The antimicrobial activity was expressed in % reduction of the organisms after contact with the test specimen compared to the number of bacterial cells surviving without any specimen or after contact with the specimen for 1 min.

The percentage reduction is calculated using the following equation.:

$$R=100(B-A)/B$$

Where:

R=% reduction
A= the surviving cells (CFU/ml) for the flasks containing the treated fabric after 1 hour;

B= "0" contact time CFU/ml for the flasks used to determine A after the addition of the treated fabric for 1 min.

Table 3. Anti-microbial activity with ASTM E 2149-01

| Fabric No. | Fabric Type | Cellulase treatment | Cutinase treatment | NaOH treatment | Reduction of ATCC 4352 bacteria (%) |
|---|---|---|---|---|---|
| 1 | TC blend | No | No | No | 17.4 |
| 2 | | buffer | buffer with X-100 | No | 4.8 |
| 3 | | 1.2 g/L Cellusoft CR | No | No | 4.8 |
| 4 | | 1.2 g/L Cellusoft CR | 0.4 mg PETHG0185/g fabric | No | 89.1 |
| 5 | 100% staple PET | No | No | No | 13.0 |
| 6 | | No | buffer with X-100 | No | 14.2 |
| 7 | | No | 0.4 mg PETHG0185/g fabric | No | 52.2 |
| 8 | | No | No | 20g/L, 100°C, 1 h | 0 |

For AATCC Test Method 100-2004 based tests, the test conditions were listed as below:
1.0 ml of an appropriate dilution of a 24 h broth culture *Klebsiella Pneumoniae* (ATCC 4352) was applied onto the circular of 100% staple PET fabric piece of 4.8 cm in diameter. The swatches were transferred aseptically to the jar. The jar tops were screwed on tightly to prevent evaporation.

Part 1 was used to calculate the bacteria recovered from the fabrics before anti-bacterial effects take place. Immediately after inoculation ("0" contact time), 100 ± 1 mL of neutralizing solution was added to jars. The jars were shaked vigorously for one minute. Make serial dilutions with water and plate (in duplicate) on nutrient agar. Dilutions of $10^0$,$10^1$,$10^2$ were usually suitable. Several different dilutions may be required for untreated control fabrics depending on the incubation period. All plates were incubated for 24-48 h at 37°C .

Part 2 was used to calculate the bacteria recovered from the fabrics after anti-bacterial effects take place. These jars containing inoculated swatches were incubated at 37°C for 24 h. After incubation, 100 ± 1 mL of neutralizing solution was added to jars. The jars were shaked vigorously for one minute. Make serial dilutions and plate (in duplicate) on nutrient agar. Dilutions of $10^0$, $10^1$, $10^2$ were usually suitable for treated test fabrics. All plates were incubated for 48 h at 37°C.

$$R=100(B-A)/B$$

Where:

R=% reduction
A= the number of bacteria recovered from the inoculated treated test specimen swatches in the jar incubated over the desired contact period (refer to part 2)
B= the number of bacteria recovered from the inoculated treated test specimen swatches in the jar immediately after inoculation at "0" contact time (refer to part 1)

Table 4. Anti-microbial activity with AATCC Test Method 100-2004

| Fabric No. | Fabric type | Cutinase treatment | NaOH treatment | Reduction of ATCC 4352 bacteria (%) |
|---|---|---|---|---|
| 6 | 100% staple PET | buffer with X-100 | No | 0 |
| 7 | | 0.4 mg PETHG0185/ g fabric | No | 96.3 |
| 8 | | No | 20g/L, 100°C, 1 h | 0 |

The test results based on both ASTM E 2149-01 and AATCC Test Method 100-2004 suggested that the cutinase treatment significantly enhanced the anti-microbial performance for the 100% PET fabric and TC blend.

**Example 4 Anti-microbial performance test by direct contact on the Agar plate**

The 100% PET fabrics before treatment (Fabric No. 5 in Example 3) and treated with cutinase (Fabric No. 7) were also tested for the anti-microbial performance on the plate.

E. coli Top10 was streaked on LB agar plate and grown at 37°C over night for single colony. The single colony was used to inoculate 3ml of LB medium in 14ml-test tube and shaking at 37°C, 200rpm for 16 hrs. The cell density was around $8x10^8$/ml. Spread 100ul of the cell culture onto the LB agar plate. The treated and untreated PET fabrics were cut into circles with 1 cm in diameter and placed on the plates. The fabric circles were lightly pressed to make sure they were tightly contacted to the plates. Then the 2 plates were incubated at 37°C for overnight. On the plate covered with untreated PET, the bacterial grew well on the whole plate, while on the plate covered with the cutinase treated fabric, E.coli only grew on the plate outside of fabric but not under the fabric. The result suggested that the cutinase treatment significantly enhanced the anti-microbial performance for PET.

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. An antimicrobial method on polyester textile by contacting polyester textile with a cutinase in an aqueous solution.

2. The method of claim 1, wherein the textile is, fabric or garment.

3. The method of claim 1 or 2, wherein the polyester is PET.

4. The method of any of the preceding claims, wherein the polyester textile is pure polyester textile or polyester/cotton blend.

5. The method of claim 1, wherein the aqueous solution further comprises one or more enzymes selected from the group consisting of lipases, esterases, laccases, peroxidases, peroxygenase and transferases.

6. The method of any of the preceding claims, wherein the cutinase is applied in the range of from about 0.01 to about 50 milligram enzyme protein per gram of polyester textile, preferably 0.05-20 milligram of enzyme protein per gram of polyester textile, more preferably 0.1-15 milligram of enzyme protein per gram of polyester textile, and even more preferably 0.2-5 milligram of enzyme protein per gram of polyester textile.

7. The method of any of the preceding claims, wherein the method is conducted in the pH range of from about pH 3 to about pH 11, preferably in the range of from about pH 4 to about pH 10, or within the range of from about pH 6 to about pH 9.

8. The method of any of the preceding claims, wherein the method is conducted in the temperature range of 40-100°C, preferably 50-90°C, preferably 60-85°C, more preferably 65-80°C, and even more preferably 70-80°C.

9. The method of any of the preceding claims, wherein the method is conducted for about 10 minutes to about 8 hours, preferably about 20 minutes to about 180 minutes, more preferably about 30 minutes to about 150 minutes, more preferably about 45 minutes to about 120 minutes.

10. The method of any of the preceding claims, wherein the antimicrobial method is to inhibit or reduce the bacterial grown on the textile.

11. The method of any of the preceding claims, wherein the treating polyester textile is manufacturing the polyester textile, especially manufacturing the polyester fabric.

12. The method of claim 11, wherein the method is in combination with any of the existing polyester fabric manufacturing steps.

## Patentansprüche

1. Antimikrobielles Verfahren auf Polyestertextil durch Inkontaktbringen von Polyestertextil mit einer Cutinase in einer wässrigen Lösung.

2. Verfahren nach Anspruch 1, wobei das Textil ein Stoff oder Kleidungsstück ist.

3. Verfahren nach Anspruch 1 oder 2, wobei der Polyester PET ist.

4. Verfahren nach einem beliebigen der voranstehenden Ansprüche, wobei das Polyestertextil reines Polyestertextil oder Polyester/Baumwollmischung ist.

5. Verfahren nach Anspruch 1, wobei die wässrige Lösung des Weiteren ein oder mehrere Enzyme umfasst, die aus der Gruppe bestehend aus Lipasen, Esterasen, Laccasen, Peroxidasen, Peroxygenase und Transferasen ausgewählt ist.

6. Verfahren nach einem beliebigen der voranstehenden Ansprüche, wobei die Cutinase im Bereich von etwa 0,01 bis etwa 50 Milligramm Enzymprotein pro Gramm Polyestertextil angewendet wird, bevorzugt 0,05-20 Milligramm Enzymprotein pro Gramm Polyestertextil, stärker bevorzugt 0,1-15 Milligramm Enzymprotein pro Gramm Polyestertextil, und sogar stärker bevorzugt 0,2-5 Milligramm Enzymprotein pro Gramm Polyestertextil.

7. Verfahren nach einem beliebigen der voranstehenden Ansprüche, wobei das Verfahren im pH-Bereich von etwa pH 3 bis etwa pH 11 durchgeführt wird, bevorzugt im Bereich von etwa pH 4 bis etwa pH 10, oder im Bereich von etwa pH 6 bis etwa pH 9.

8. Verfahren nach einem beliebigen der voranstehenden Ansprüche, wobei das Verfahren im Temperaturbereich von 40-100°C, bevorzugt 50-90°C, bevorzugt 60-85°C, stärker bevorzugt 65-80°C, und sogar stärker bevorzugt 70-80°C durchgeführt wird.

9. Verfahren nach einem beliebigen der voranstehenden Ansprüche, wobei das Vefahren für etwa 10 Minuten bis etwa 8 Stunden, bevorzugt etwa 20 Minuten bis etwa 180 Minuten, stärker bevorzugt etwa 30 Minuten bis etwa 150 Minuten, stärker bevorzugt etwa 45 Minuten bis etwa 120 Minuten durchgeführt wird.

10. Verfahren nach einem beliebigen der voranstehenden Ansprüche, wobei das antimikrobielle Verfahren darin besteht, das bakterielle Wachstum auf dem Textil zu inhibieren oder verringern.

11. Verfahren nach einem beliebigen der voranstehenden Ansprüche, wobei das Behandeln von Polyestertextil Herstellen des Polyestertextils ist, insbesondere Herstellen des Polyesterstoffes.

12. Verfahren nach Anspruch 11, wobei das Verfahren in Kombination mit einem beliebigen der bestehenden Polyesterstoff-Herstellungsschritte ist.

## Revendications

1. Méthode antimicrobienne sur un textile en polyester par mise en contact du textile en polyester avec une cutinase dans une solution aqueuse.

2. Méthode selon la revendication 1, dans lequel le textile est une étoffe ou un habit.

3. Méthode selon la revendication 1 ou 2, dans laquelle le polyester est le PET.

4. Méthode selon l'une quelconque des revendications précédentes, dans laquelle le textile en polyester est un textile en polyester pur ou un mélange de polyester/coton.

5. Méthode selon la revendication 1, dans laquelle la solution aqueuse comprend en outre une ou plusieurs enzymes choisies dans le groupe constitué par les lipases, les estérases, les laccases, les peroxydases, les peroxygénases et les transférases.

6. Méthode selon l'une quelconque des revendications précédentes, dans laquelle la cutinase est appliquée à raison d'environ 0,01 à environ 50 milligrammes de protéine enzymatique par gramme de textile en polyester, de préférence de 0,05 à 20 milligrammes de protéine enzymatique par gramme de textile en polyester, mieux encore de 0,1 à 15 milligrammes de protéine enzymatique par gramme de textile en polyester, et plus particulièrement de 0,2 à 5 milligrammes de protéine enzymatique par gramme de textile en polyester.

7. Méthode selon l'une quelconque des revendications précédentes, laquelle méthode est mise en oeuvre à un pH situé dans la plage allant d'environ 3 à environ 11, de préférence dans la plage allant d'environ 4 à environ 10, ou dans la plage allant d'environ 6 à environ 9.

8. Méthode selon l'une quelconque des revendications précédentes, laquelle méthode est mise en oeuvre à une température située dans la plage allant de 40 à 100 °C, de préférence de 50 à 90 °C, de préférence de 60 à 85 °C, mieux encore de 65 à 80 °C, et plus particulièrement de 70 à 80 °C.

9. Méthode selon l'une quelconque des revendications précédentes, laquelle méthode est mise en oeuvre pendant environ 10 minutes à environ 8 heures, de préférence environ 20 minutes à environ 180 minutes, plus particulièrement environ 30 minutes à environ 150 minutes, plus particulièrement environ 45 minutes à environ 120 minutes.

10. Méthode selon l'une quelconque des revendications précédentes, laquelle méthode antimicrobienne a pour but d'inhiber ou de réduire la croissance bactérienne sur le textile.

11. Méthode selon l'une quelconque des revendications précédentes, dans laquelle le traitement du textile en polyester est la fabrication du textile en polyester, en particulier la fabrication de l'étoffe en polyester.

12. Méthode selon la revendication 11, laquelle méthode est combinée avec l'une quelconque des étapes existantes de fabrication d'une étoffe en polyester.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1999001604 A
- WO 2001092502 A
- WO 2014012506 A
- US 6815190 B
- US 20020066144 A
- EP 2476798 A
- US 5827719 A
- WO 9009446 A
- WO 9414964 A
- WO 9403578 A
- WO 10107560 A
- US 5389536 A
- WO 0034450 A
- US 20040171154 A
- WO 9517413 A
- WO 9522625 A
- US 5223409 A
- WO 9206204 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 2007087508 A
- WO 2009109500 A
- WO 9324618 A
- WO 9510602 A
- WO 9815257 A
- WO 2008119780 A
- WO 9629397 A

### Non-patent literature cited in the description


- *Journal of Applied Polymer Science,* 2009, vol. 112, 1927-1932
- *Textile Research Journal,* vol. 78 (1), 60-72
- Recommendations of the Nomenclature Committee of the International Union of Biochemistry and Molecular Biology. Academic Press Inc, 1992
- **SCHERER ; DAVIS.** *Proc. Natl. Acad. Sci. USA,* 1979, vol. 76, 4949-4955
- **BARTON et al.** *Nucleic Acids Res.,* 1990, vol. 18, 7349-4966
- **STORICI et al.** *Nature Biotechnol.,* 2001, vol. 19, 773-776
- **KREN et al.** *Nat. Med.,* 1998, vol. 4, 285-290
- **CALISSANO ; MACINO.** *Fungal Genet. Newslett.,* 1996, vol. 43, 15-16
- **TIAN et al.** *Nature,* 2004, vol. 432, 1050-1054
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE.** *EMBOSS: The European Molecular Biology Open Software Suite,* 2000
- **HENRISSAT B.** *Biochem. J.,* 1991, vol. 280, 309-316
- **HENRISSAT B. ; BAIROCH A.** *Biochem. J.,* 1996, vol. 316, 695-696
- **DARTOIS et al.** *Biochemica et Biophysica Acta,* 1993, vol. 1131, 253-360